# EUROPEAN PATENT SPECIFICATION

(11) **EP 3 337 309 B1**
(45) Date of publication and mention of the grant of the patent: **02.10.2024**
(21) Application number: 16762738.9
(22) Date of filing: 18.08.2016
(51) Int. Cl.: A01G 7/06

(54) **METHOD FOR REDUCING PATHOGENS**
VERFAHREN ZUR ERREGERREDUZIERUNG
PROCÉDÉ DE RÉDUCTION DE PATHOGÈNES

(30) Priority: 18.08.2015 NL 2015316
(43) Date of publication of application: 27.06.2018
(73) Proprietor: Rede Investments BV, 6681 TS Bemmel (NL)
(72) Inventor: VAN ONNA, Gerardus Bernardus Johannus, 6681 TS Bemmel (NL); DERKSEN, Marc Alexander, 2807 HX Gouda (NL)
(74) Representative: Arnold & Siedsma
(86) International application number: PCT/EP2016/069642
(87) International publication number: WO 2017/029373

(56) References cited:
- WO-A1-2006/021225
- WO-A1-2010/066733
- WO-A2-2014/001476
- JP-A- 2013 230 122
- US-A1- 2012 210 637

## Description

The present invention relates to a method for treating an infection by *Pseudomonas syringae* and/or *Pseudomonas syringae pv actinidiae* in a tree.

Many trees suffer from pests and/or infections from which they ultimately die. This is a major burden on the organisations and authorities responsible for the maintenance of parks and public plantations, on (professional) horticulturists, as well as on individuals keeping trees in their gardens and backyards, as the dead trees need to be cut down, removed and replaced with new trees. Yields of fruit trees can be completely lost.

An example of an infection is for instance the infection of horse chestnut trees and/or kiwifruit trees by the bacterial pathogen, *Pseudomonas syringae* respectively *Pseudomonas syringae pv actinidiae.* This pathogen infects the bark around the trunk and main branches of the tree, and leads to the disease named canker (bleeding canker in horse chestnut trees).

At the site of infection the tissue dies and the bark peels away. This allows a sticky, blood coloured liquid to ooze from blemishes on the bark of infected trunks, leaving rust spots behind when it dries up. The oozing liquid is clear yellow at first, then becomes dark, blood coloured and sticky, and runs down the trunk. Eventually the tree dies.

Chances of survival of an affected tree are slim: no full recovery has ever been reported. Normally, it takes between 1 to 3 years from the appearance of the first symptoms until the tree dies. However, sometimes an affected tree dies within a very short period of time such as a couple of months.

It is not possible to identify (bleeding) canker in an early stage, as the infection occurs underneath the bark. The rust spots that appear on the bark of the tree are initially too small to be identified. These are only noticed when they gradually become larger and start bleeding. In addition, no other symptoms have been specifically correlated with the disease. The yellowing or browning of leaves, dying twigs, and/or drying of fruits are all symptoms that may also have a different cause. It is therefore that most trees are already infected and sick before showing any visual symptoms.

The bacterial pathogen *Pseudomonas syringae* multiplies in the cadmium tissue of the bark, resulting in the degradation of the bark and subsequent bleeding. Laboratory tests have shown that the multiplication of the bacteria can be inhibited by exposing the bacteria to a temperature of at least 39°C. This indicates that (bleeding) canker may be treated by exposing the bacteria in the bark of infected trees to temperatures of at least 39°C.

One way to use heat treatment for reducing or eliminating bacterial growth is by creating a climate chamber or by arranging a garden hose around the trunk or branches of the tree at the site of infection and passing hot water through the hose. The hose-wrapped trunk and/or branches of the tree need to be covered with insulation material in order to avoid heat loss as good as possible. It has been demonstrated that bark samples of affected horse chestnut trees contained *Pseudomonas syringae* before such heat treatment and no longer contained *Pseudomonas syringae* after such heat treatment. Such a treatment is detailed in WO 2014/001476 A2.

The above described methods are however not very practical and for many trees even impossible if they contain irregularities. In order to heat the bark of a tree, more than 250 up to 1.000 meters of hose would be required. Water cools down every meter. It is therefore not feasible to heat the entire bark to 39 °C. Especially not since this method greatly depends on the temperature of the groundwater used and the outside air temperature. A major complication is that the temperature of the groundwater and outside temperature vary a lot during night and day. Also, this water hose method takes a very long time (up to 72 hours).

Furthermore, exposing a tree to high temperatures for a long period of time may harm the tree.

It is therefore an object of the present invention to provide a practical method for treating a pest and/or infection in an affected subject, such as for example treating bleeding canker in an affected horse chestnut tree and/or treating canker in an affected kiwifruit tree.

It is a further object of the present invention to provide an efficient method for the disinfection, pasteurisation and/or sterilisation of an article.

These objects are met by providing a method wherein the subject is exposed to infrared radiation.

During the research leading to the present invention, the inventors found that the method of the present invention is very useful for disinfecting articles, such as seeds, pollen, soil, flowers, vegetables, and fruits and even for the pasteurisation and sterilisation of other foodstuff, such as milk. Also (storage) containers, such as (silo's) may be disinfected by the method of the present invention. The present methods for disinfecting these and similar articles all have certain disadvantages. For instance, in the process of disinfection part of the seeds lose their viability and may cause the occurrence of rotting processes. The quantity, as well as the quality, of disinfected seeds thus needs to be improved. The same applies to flowers, vegetables and fruits. The present methods for pasteurisation and sterilisation of foodstuff, such as dairy products, are not very efficient, as they take a long time and are mostly unhygienic. They are further not very accurate as the temperature during the process cannot be regulated.

The invention thus relates to a method for treating an infection in a tree according to claim 1.

This method is quick and can be highly accurately controlled.

The subject may be any subject, such as for instance a plant, a tree, a piece of furniture, a part of a building, and other objects. The subject is not a human being or an animal.

The energy or generated heat of infrared radiation is absorbed by the subject and heats up the subject. This heat reduces or eliminates the pathogens, such as bacteria, fungi, and/or parasites, that are present in and/or on the subject.

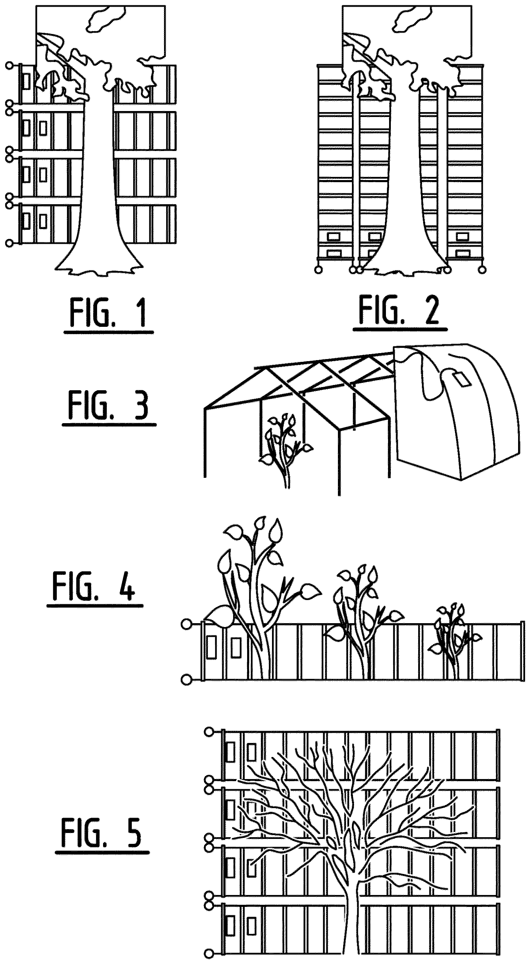

In one embodiment, the subject is exposed to infrared radiation by applying a source of infrared radiation to the subject, preferably at the site of infection, and/or by positioning the source of the infrared radiation in the vicinity of the subject. The source of the infrared radiation can be applied to, and/or positioned in the vicinity of, the subject in any suitable manner. A few examples are given in Figures 1-5. For instance, the source of the infrared radiation may be wrapped around part(s) of the subject, and/or positioned in the vicinity of the subject, such as for instance in front of and/or behind and/or on the sides and/or above part(s) of, or the entire, subject. It may also be positioned to cover the entire subject.

The inventors have found that a carbon crystal electric heating element is particularly useful as the source of infrared radiation. Carbon crystal electric heating is a technology used in the heating of buildings, such as for instance in floor and wall heating systems. It produces far infrared radiation that does not heat the air but heats the object. A carbon crystal heating element heats up in a couple of seconds and is capable of distributing the heat evenly to a large surface with high accuracies, even when the surface has irregularities. It is therefore very suitable for evenly heating, for instance, the trunk and/or branches of a tree.

The carbon crystal electric heating element may be composed of a foil comprising carbon crystals. "Brownian motion" is initiated by providing an electric current, wherein the friction and impact of the carbon molecules generate heat and infrared radiation. The conversion rate obtained by converting electricity into infrared radiation can be as high as 96%, making it a very efficient way of heating.

The carbon crystal heating element may also be composed of a fabric comprising carbon crystals. The manner in which this fabric is woven and the manner wherein the carbon crystals are present in this fabric determine the wavelength of the infrared radiation. Wavelengths from 2 up to 4 UM may be achieved. Carbon crystal electric heating elements generating a particular wavelength are commercially available. Different wavelengths kill different pathogens. By tuning the wavelength in the carbon crystal electric heating element it is possible to optimize the killing of different pathogens more efficiently.

The carbon crystal electric heating element needs to be set at a temperature sufficient to heat up the subject to the temperature at which the bacteria, fungi, and/or parasites are killed. In the invention, the subject is exposed to a temperature of at least 39 °C. In order for the cambium of a tree to reach a temperature of about 39°C within a reasonable period of time, the carbon crystal electric heating element needs to be applied to the tree and set at at least 60-70°C. A carbon crystal electric heating element can, however, be set in the range of 0-140°C, depending on its application. It may even reach temperatures as high as 3600 °C.

It may take some time before the subject has reached the desired temperature. For example, it takes about 180 minutes for a tree to reach about 39°C when a carbon crystal electric heating element is set at about 60-70 and 120 minutes when set at about 100°C. This is, however, much faster than when using a climate chamber or water hose. The time and temperature settings can easily be adapted to the circumstances, such as for instance to the type of bacteria, fungi, and/or parasites that needs to be killed and the outdoor conditions. The carbon crystal electric heating element needs to be applied to and/or positioned in the vicinity of the subject for a period sufficient to kill all, or nearly all, bacteria, fungi, and/or parasites. It is, however, important that the tree is not exposed to a high temperature for too long, as high temperatures affect the health of a tree. In some circumstances it may be better to increase the temperature above the temperature required to kill the pathogens in order to reduce the time that the subject needs to be exposed to the high temperature. It may for instance be better to heat up the bark of a tree to about 45 °C for a period of about 15 minutes instead of heating up the bark to about 39 °C for a period of about 30 minutes.

In the invention, the subject is exposed to the infrared radiation for less than 48 hours, in preferred embodiments, the subject is exposed to the infrared radiation for preferably less than 24 hours, more preferably less than 12 hours, and most preferably between 2-12 hours.

It has been demonstrated that for some applications bacterial growth can be eliminated within the time needed for the subject to reach the temperature sufficient to kill the bacteria. It was not required to expose the bacteria for a longer time to this temperature.

One of the main advantages of the use of a carbon crystal electric heating element in the method(s) of the present invention is that it is very easy to use. It can be produced in every shape and flexibility as required by the intended application, and is relatively cheap. As it is light, it is particular suitable for the treatment of pests and/or infections of trees.

In a preferred embodiment, the method further comprises monitoring and/or regulating the temperature of the affected subject with a temperature sensor. The temperature sensor is applied into a hole created in the wood of the affected subject. The temperature sensor is coupled to the source of the infrared radiation. When coupled, the source of the infrared radiation is switched off when the temperature of the tree reaches the desired temperature and/or a temperature which is considered harmful to the tree. Also, the temperature of the tree can be held at a set temperature for a longer period of time, by reducing the current applied to the carbon crystal electric heating element. The source of infrared radiation is turned back on when the temperature of the affected subject drops below the desired temperature.

The method of the present invention can be used to treat all types of pests and/or infections of a variety of subjects, as long as the temperature of the affected subject reaches the temperature at which the bacteria, fungi, and/or parasites are killed. The temperatures at which bacteria, fungi, parasites, etc are killed are well known. For example, the temperature required for killing *Pseudomanas syringae* and/or *Pseudomonas syringae pv actinidiae* is 39 °C.

The present invention thus also relates to the method of the present invention for treating a pest and/or infection in an affected subject, to the use of a carbon crystal electric heating element for treating a pest and/or infection in an affected subject, and to a method for treating a pest and/or infection in an affected subject comprising exposing the affected subject to infrared radiation, in particular infrared radiation from a carbon crystal electric heating element.

The subject may be any subject that is affected by the presence of pathogens in and/or on the subject. The subject may for instance be a plant, a tree, a piece of furniture, a part of a building, and other objects. The subject is not a human being or an animal.

In the invention, the affected subject is a tree, in particular a tree of a species selected from the group consisting of *Aesculus,* in particular *Aesculus hippocastanum, Actinidia,* in particular *Actinidia chinesis* and/or *Actinidia Deliciosa.* In other embodiments, species may be selected from the group consisting of *Arecaceae, Picea sitchensis, Picea pungens, Dilleniaceae, Sanryoku, Prunus, Pyrus and Pyrus communis, Citrus, Malus, Catalpa bignonioides, Magnolia, Oleaceae Quercus robur, Platanus x hispanica, Ailanthus altissima, Ailanthus altissima, Carpinus betulus, Salix alba, Ulmus x hollandica, Quercus robur, Styphnolobium japonicum, Pterocarya fraxinifolia, Tilia x europaea, Castanea sativa, Ginkgo biloba, Salix alba, Betula pendula, Acer pseudoplatanus 'Leopoldii', Fagus sylvatica 'Atropunicea', Platanus orientalis, Alnus cordata, Populus x Canadensis, Fagus sylvatica, Pyrus communis, Prunus serrulata, Alnus glutinosa, Juglans regia, Taxodium ascendens, Tilia tomentosa, Liriodendron tulipifera, Quercus rubra, Sequoiadendron giganteum, Castanea sativa, Acer pseudoplatanus, Picea pungens, Quercus robur, Fraxinus excelsior, Vitis vinifera, Pyrus communis, Populus nigra, rataegus laevigata, Fagus sylvatica, deliciosa, Vaccinium, Ficus, Prunus, Malus domestica, Pyrus communis, Pagaceae, Rosaceae, Oleaceae, Malus* and *Castanea.*

In one embodiment, the pest is caused by an organism selected from the group consisting of *Rhynchophorusferrugineus, Synanthedon myopaeformis, Tuta absoluta* , *Ardis brunniventris, Parthenolecanium cornii, Thaumetoppoea processionea, Lygocoris pabulinus, Archips rosana, Stephanitis, Argyresthia trifasciata, Cameraria ohridella, Resseliella oculiperda, Agrilus sinuatus, Saperda carcharias, Stigmella pupulnea, Oligonychus unuguis, Cryptorrhynchus laphatii, Cossus cossus, Xestobium rufovillosum, Cerambycidae, sapwood beetle, Scolitus multistriatus, Anoplophora chinensis,* and *Elatobium abietinum.*

In one embodiment, the infection is an infection caused by bacteria, fungi, and/or parasites.

In the invention, the bacteria is selected from the group consisting of *Pseudomonas syringae* and/or *Pseudomonas syringae pv actinidiae*

In other embodiments, bacteria may be selected from the group consisting of *Pseudomonas syringae subsp savastonii, Xanthomonas, Erwinia,* in particular *Erwinia amylovora, Erwinia carotovora sub.sp Atroseptica, Erwinia carotovora sub.sp carotovor a., Xanthomonas* in particular *Xanthomonas arboricola pv pruni, Vaccinium corymbosum,* and *Xylella,* in particular *Xylella fastidiosa Tobacco Ringspot Virus.*

The fungi, which include yeast, may be selected from the group consisting of *Verticillium dahlia, Phytophthora* in particular *Phytophthora ramorum* and *Phythophthora cactorum, Ceratocystis fimbriata, Cylindrocarpon mali, Chalaropsis thielavioides, Chalara fraxinea, Fusarium foetens, Splanchnonema platani, Phytophthora ramorum, Guignardia aesculi, Gnomonia leptostyla or marssonina, Cylindrocladium buxicola, Hymenoscyphus pseudoalbidus, Taphrina betulina, Splanchnonema platani, Nectria cinnabarina, Gymnosporangium clavariiforme, Venturaria inaequalis, Venturia, Didymascella thujina, Kabatina juniperi* or *Didymascella thujae, Nectria galligena, Ascomycetes,* and *Fusarium oxysporum.*

The parasites may be woodworm.

In one embodiment, the method of the present invention is used for the treatment of an *Aesculus,* in particular an *Aesculus hippocastanum,* and/or an *Actinidia,* in particular an *Actinidia chinesis* and/or an *Actinidia deliciosa,* tree infected with *Pseudomonas syringae* and/or *Pseudomonas syringae pv actinidiae* respectively, wherein the temperature of the source of the infrared radiation is set at a temperature of between 50-80 °C, and preferably between 60-70°C. In this embodiment, it takes about 180 minutes to completely eliminate the pathogens in the tree.

In another embodiment, the method of the present invention is used for the treatment of an *Aesculus,* in particular an *Aesculus hippocastanum,* and/or an *Actinidia,* in particular an *Actinidia chinesis* and/or an *Actinidia deliciosa,* tree infected with *Pseudomonas syringae* and/or *Pseudomonas syringae pv actinidiae* respectively, wherein the temperature of the source of the infrared radiation is set at a temperature of between 80-120 °C, and preferably at about 100 °C. In this embodiment, it takes about 70-80 minutes to completely eliminate the pathogens in the tree.

The method of the present invention may also be used in applications other than the treatment of trees. One such application is the treatment of with woodworm infected furniture. The source of the infrared radiation, such as a carbon crystal heating element, can be applied to and/or positioned in the vicinity of the furniture in a similar way as in the treatment of trees.

The present inventors have further found that the method of the present invention can be used in the disinfection of seeds, pollen, soil, flowers, vegetables, fruits and the like. The present invention therefore also relates to the use of the method of the invention for the disinfection of an article, to the use of a carbon crystal electric heating element for the disinfection of an article, and to a method for disinfecting an article comprising exposing an article to infrared radiation, in particular infrared radiation from a carbon crystal electric heating element. Examples of an article include, but are not limited to, a seed, pollen, soil, flower, vegetable and fruit.

The method of the present invention may also be applied on other foodstuff, such as dairy products. The present invention thus also relates to the use of the method of the invention for the pasteurisation or sterilisation of foodstuff, to the use of a carbon crystal electric heating element for the pasteurisation or sterilisation of foodstuff, and to a method for pasteurising or sterilising foodstuff comprising exposing the foodstuff to infrared radiation, in particular infrared radiation from a carbon crystal electric heating element.

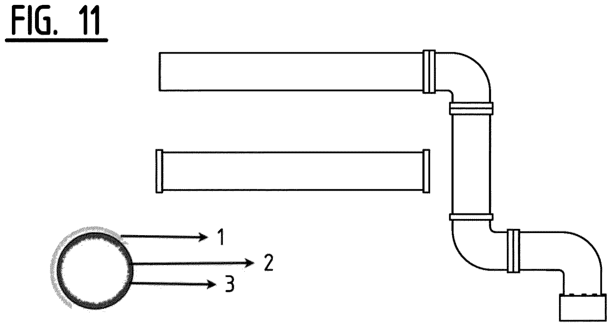

The disinfection of an article may be achieved by placing the article in a chamber comprising the source of infrared radiation. For instance, one or more walls of the chamber may be entirely or partly composed of a carbon crystal heating element. For example, a pipe comprising a lining of carbon crystal electric heating element as depicted in figure 11 may be used. In this embodiment the article to be disinfected, or pasteurised or sterilised, is fed into the pipe where it is heated to the desired temperature by the carbon crystal heating element. The article to be disinfected, such as pollen and seeds, may be blown through the pipe or may be fed into the pipe as (part of) a liquid. Because the carbon crystal heating element is capable of heating up to very high temperatures in a very short period of time, pathogens in and/or on the article will be killed before the article reaches the end of the pipe. The length of the pipe may be adjusted to suit its purpose. For instance, the pipe may be longer if more time is required to reach the desired temperature or to keep the article at the desired temperature for a longer period of time. Passing an article through a device comprising a carbon crystal heating element allows for high-throughput disinfection of articles.

All applications wherein the killing of bacteria, fungi, and/or parasites is desired will benefit from the present invention.

### FIGURES

Figure 1 is an illustration showing how a carbon crystal heating element can be wrapped around a tree horizontally.
Figure 2 is an illustration showing how a carbon crystal heating element can be wrapped around a tree vertically.
Figure 3 is an illustration showing how a carbon crystal heating element can be used as a surface covering an area with one or more trees.
Figure 4 is an illustration how a carbon crystal heating element can be wrapped horizontally around multiple trees.
Figure 5 is an illustration how an entire tree is covered with a high shield of carbon crystal heating element.
Figure 6A is a graph showing the temperature of the tree as measured by a sensor placed in the bark of the tree.
Figure 6B is a table showing the presence of *Pseudomonas syringae* in a tree after treatment with a carbon crystal heating element.
   0 = not detected
   1 = very slightly present
   2 = slightly present
   3 = very moderately present
   4 = moderately present
   5 = strongly present
   6 = very strongly present
Figure 7 is a table showing the presence of *Erwinia carotovora subsp. carotovora* in a tree after treatment with a carbon crystal heating element.
   0 = not detected
   1 = very slightly present
   2 = slightly present
   3 = very moderately present
   4 = moderately present
   5 = strongly present
   6 = very strongly present
Figure 8 shows 6 petri dishes. The upper left petri dish is the positive control, the lower left petri dish is the negative control. The other petri dishes are infected and subsequently treated with a carbon crystal heating element for a period of 0 minutes (upper middle), 10 minutes (upper right), 20 minutes (lower middle) and 30 minutes (lower right).
Figure 9 shows 6 petri dishes. The upper left petri dish is the positive control, the lower left petri dish is the negative control. The other petri dishes are infected and subsequently treated with a carbon crystal heating element for a period of 0 minutes (upper middle), 10 minutes (upper right), 20 minutes (lower middle) and 30 minutes (lower right).
Figure 10 shows 7 tubes. Tube 1 is the positive control, tube 2 the negative control. The other tubes contain infected swabs and are treated with a carbon crystal electric heating element for a period of 0 minutes (tube 3), 10 minutes (tube 4), 20 minutes (tube 5) and 30 minutes (tube 6). Tube 7 is a negative control exposed to the carbon crystal heating element for 30 minutes.
Figure 11 shows an example of a pipe (2) comprising insulation material on the outer side (1) and a lining of carbon crystal heating element on the inner side (3).

### EXAMPLE 1

1-3.5 m long stretches of carbon crystal electric heating material were covered with a fabric provided with insulation materials on the outer side. These stretches were applied both horizontally and vertically to the trunk of a horse chestnut tree. The stretches were applied such that they covered the trunk and/or some bigger branches.

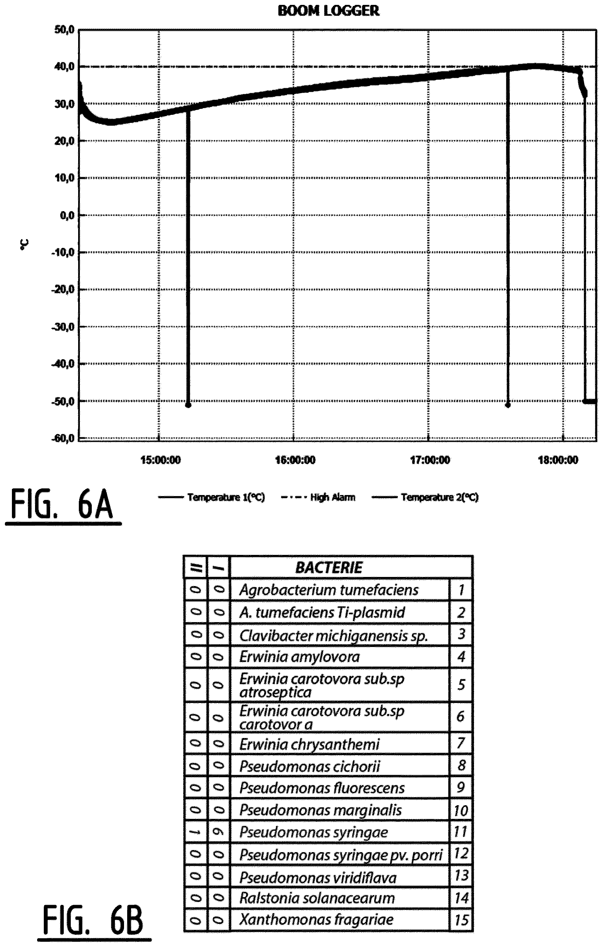

A 220V electric current was applied. The carbon crystal electric heating material was heated to approximately 60-67 °C. Figure 6A shows the temperature of the tree from the start until the end of the treatment.

After approximately 180 minutes, the bark of the tree reached 40 °C as measured by the sensors applied into small holes prepared in the bark before applying the carbon crystal electric heating material.

Samples of the bark were taken before (monster I) and after (monster II) treatment and analysed. The results in Figure 6B indicate that *Pseudomonas syringae* was very strongly present in the sample taken before the treatment, whereas *Pseudomonas syringae* was only very slightly present in the sample taken after the treatment. These results may indicate that *Pseudomonas syringae* is still slightly present after the treatment, however, this may also be due to the presence of remaining DNA and not to the actual presence of live bacteria.

### EXAMPLE 2

The experiment as described in Example 1 was repeated in order to determine the efficacy of the method of the present invention in reducing *Erwinia carotovora subsp. carotovora* growth in a horse chestnut tree.

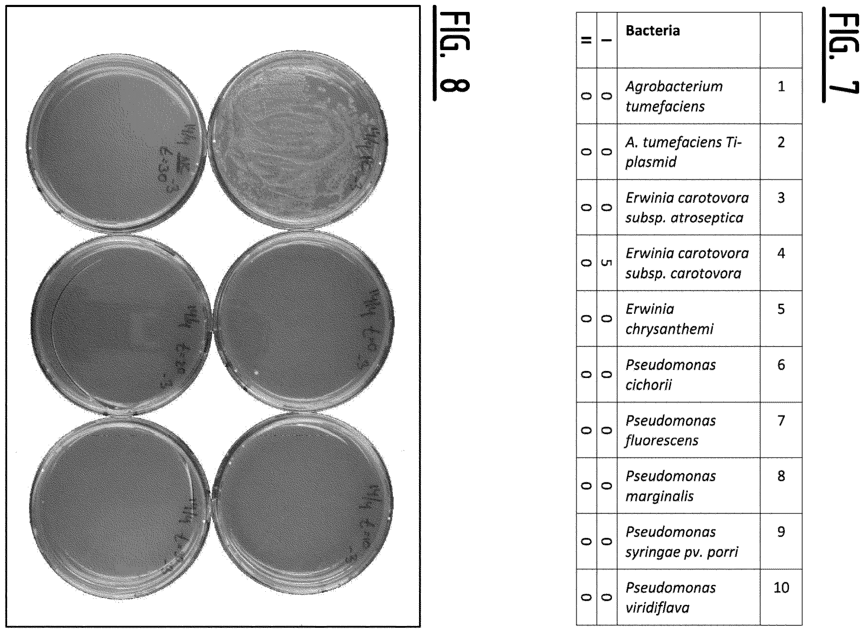

In the sample taken before treatment with the carbon crystal electric heating element *Erwinia carotovora subsp. carotovora* was strongly present, whereas *Erwinia carotovora subsp. carotovora* was no longer detected in the sample after treatment (see Figure 7).

### EXAMPLE 3

Petri dishes were infected with *Pseudomonas syringae pv actinidiae* and exposed to infrared radiation from a carbon crystal heating element. The positive control was infected but not exposed to the carbon crystal electric heating element. The negative control was not infected but was exposed to the carbon crystal electric heating element for 30 minutes.

The petri dishes were heated to 50°C. At t=0, which is the temperature at which the carbon crystal heating element reached 50°C, the first petri dish was removed. The other petri dishes were removed after 10 (t=10), 20 (t=20) and 30 minutes (t=30) of exposure to the carbon crystal electric heating element.

As shown in Figure 8, no bacteria developed on the petri dish removed at t=0, indicating that the time required for heating up the carbon crystal heating element is sufficient to kill *Pseudomonas syringae pv actinidiae.* The petri dishes removed at the other time points did also not show any bacterial growth.

### EXAMPLE 4

Petri dishes were infected with *Xylella fastidosa* and exposed to infrared radiation from a carbon crystal heating element. The positive control was infected but not exposed to the carbon crystal electric heating element. The negative control was not infected but was exposed to the carbon crystal electric heating element for 30 minutes.

The petri dishes were heated to 55°C.

At t=0, which is the temperature at which the carbon crystal heating element reached 50°C, the first petri dish was removed. The other petri dishes were removed after 10 (t=10), 20 (t=20) and 30 minutes (t=30) of exposure to the carbon crystal electric heating element.

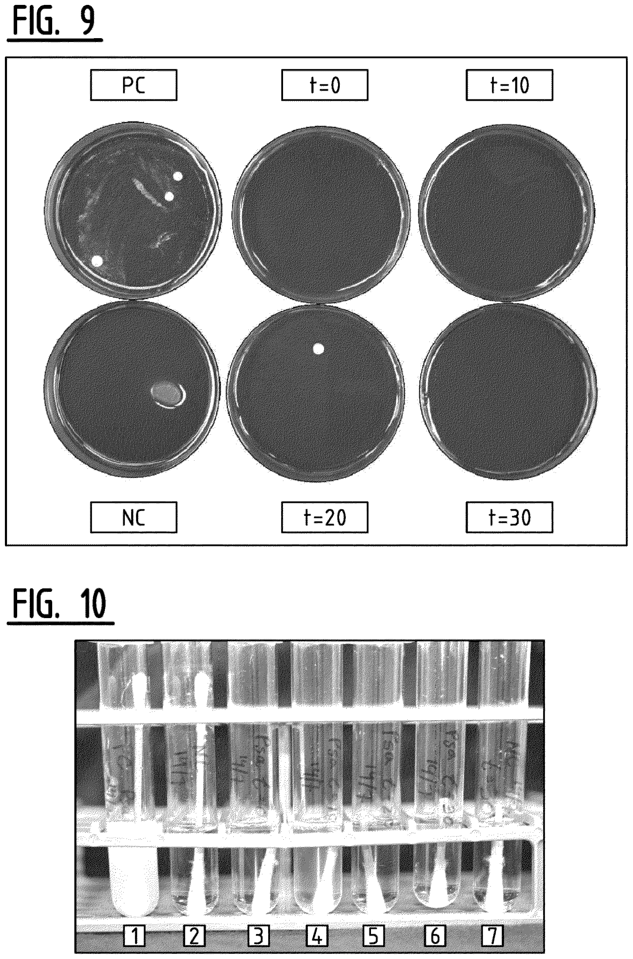

As shown in Figure 9, no bacteria developed on this petri dish removed at t=0, indicating that the time required for heating up the carbon crystal heating element is sufficient to kill *Xylella fastidosa.* The petri dishes removed at the other time points did also not show any bacterial growth, except for t=20.

### EXAMPLE 5

Swabs infected with *Pseudomonas syringae pv actinidiae* were placed in tubes containing medium and exposed to infrared radiation from a carbon crystal heating element. The positive control was infected but not exposed to the carbon crystal electric heating element. One negative control was not infected and not exposed to the carbon crystal electric heating element (tube 2) and one negative control was not infected but was exposed to the carbon crystal electric heating element (tube 7) for 30 minutes.

The tubes were heated to 50°C. At t=0, which is the temperature at which the carbon crystal heating element reached 50°C, the first tube was removed. The other tubes were removed after 10 (t=10), 20 (t=20) and 30 minutes (t=30) of exposure to the carbon crystal electric heating element.

As shown in Figure 10, no bacteria developed in the tube removed at t=0, indicating that the time required for heating up the carbon crystal heating element is sufficient to kill *Pseudomonas syringae pv actinidiae.* The tubes removed at the other time points did also not show any bacterial growth, except for tube 4.

### EXAMPLE 6

The experiment as described in Example 1 was repeated with different settings. The carbon crystal electric heating element was set at about 100 °C. It took about 70-80 minutes for the carbon crystal electric heating element to heat up to this temperature. A sample taken from the tree after 120 minutes exposure to the carbon crystal electric heating element showed no signs of pathogens.

## Claims

1. A method for treating an infection by *Pseudomonas syringae* and/or *Pseudomonas syringae pv actinidiae* in a tree, comprising:
applying a temperature sensor in a hole created in a wood of the tree;
arranging a carbon crystal electric heating element in a vicinity of the tree; and coupling the temperature sensor to the carbon crystal electric heating element; and
maintaining the temperature to be within a temperature range for a period less than 48 hours comprising:
heating the tree using infrared light emitted by the carbon crystal electric heating element;
measuring a temperature of the tree using the temperature sensor;
switching off the carbon crystal electric heating element when the measured temperature has reached a desired temperature; and
switching the carbon crystal electric heating element back on when the measured temperature drops below the desired temperature;
wherein the desired temperature is at least 39 degrees Celsius; and
wherein the tree is a tree of the genus Aesculus, in particular a tree of the species Aesculus hippocastanum, and/or a tree of the genus Actinidia, in particular a tree of the species Actinidia chinesis and/or Actinidia deliciosa.

2. The method according to claim 1, wherein the carbon crystal electric heating element is composed of a foil comprising carbon crystals, wherein the carbon crystal electric heating element is configured to generate heat and infrared radiation when an electric current is provided thereto.

3. The method according to claim 1, wherein the carbon crystal electric heating element is composed of a woven fabric comprising carbon crystals, wherein the carbon crystal electric heating element is configured to generate infrared radiation having a wavelength from 2 up to 4 micrometer.

4. The method according to any of the previous claims, wherein said arranging a carbon crystal electric heating element in a vicinity of the tree comprises wrapping the carbon crystal electric heating element around part(s) of the tree.

5. The method according to any of the previous claims, wherein the period during which the temperature is maintained to be within a temperature range is less than 24 hours, more preferably less than 12 hours, and most preferably between 2-12 hours.

## Patentansprüche

1. Verfahren zur Behandlung einer Infektion durch *Pseudomonas syringae* und/oder *Pseudomonas syringae pv actinidiae* in einem Baum, umfassend:
Anbringen eines Temperatursensors in einem im Holz des Baumes geschaffenen Loch;
Anordnen eines elektrischen Kohlenstoffkristall-Heizelements in der Nähe des Baumes; und Koppeln des Temperatursensors mit dem elektrischen Kohlenstoffkristall-Heizelement; und
Halten der Temperatur innerhalb eines Temperaturbereichs für einen Zeitraum von weniger als 48 Stunden, umfassend:
Erwärmen des Baumes mithilfe von Infrarotlicht, das vom elektrischen Kohlenstoffkristall-Heizelement ausgestrahlt wird;
Messen einer Temperatur des Baums mithilfe des Temperatursensors;
Ausschalten des elektrischen Kohlenstoffkristall-Heizelements, wenn die gemessene Temperatur eine gewünschte Temperatur erreicht hat; und
erneutes Einschalten des elektrischen Kohlenstoffkristall-Heizelements, wenn die gemessene Temperatur unter die gewünschte Temperatur fällt;
wobei die gewünschte Temperatur mindestens 39 Grad Celsius beträgt; und
wobei der Baum ein Baum der Gattung Aesculus, insbesondere ein Baum der Art Aesculus hippocastanum, und/oder ein Baum der Gattung Actinidia, insbesondere ein Baum der Art Actinidia chinesis und/oder Actinidia deliciosa, ist.

2. Verfahren nach Anspruch 1, wobei das elektrische Kohlenstoffkristall-Heizelement aus einer Folie besteht, die Kohlenstoffkristalle umfasst, wobei das elektrische Kohlenstoffkristall-Heizelement so konfiguriert ist, dass es Wärme und Infrarotstrahlung erzeugt, wenn ihm ein elektrischer Strom zugeführt wird.

3. Verfahren nach Anspruch 1, wobei das elektrische Kohlenstoffkristall-Heizelement aus einem Gewebe besteht, das Kohlenstoffkristalle umfasst, wobei das elektrische Kohlenstoffkristall-Heizelement konfiguriert ist, um Infrarotstrahlung mit einer Wellenlänge von 2 bis 4 Mikrometer zu erzeugen.

4. Verfahren nach einem der vorstehenden Ansprüche, wobei das Anordnen eines elektrischen Kohlenstoffkristall-Heizelements in der Nähe des Baums das Wickeln des elektrischen Kohlenstoffkristall-Heizelements um einen Teil oder Teile des Baumes umfasst.

5. Verfahren nach einem der vorstehenden Ansprüche, wobei der Zeitraum, während dem die Temperatur innerhalb eines Temperaturbereichs gehalten wird, weniger als 24 Stunden, mehr bevorzugt weniger als 12 Stunden und am meisten bevorzugt zwischen 2 und 12 Stunden beträgt.

## Revendications

1. Procédé permettant de traiter une infection par *Pseudomonas syringae* et/ou *Pseudomonas syringae pv actinidiae* dans un arbre, comprenant :
l'application d'un capteur de température dans un trou créé dans un bois de l'arbre ;
l'agencement d'un élément chauffant électrique à cristaux de carbone dans un voisinage de l'arbre ; et le couplage du capteur de température à l'élément chauffant électrique à cristaux de carbone ; et
le maintien de la température pour qu'elle soit au sein d'une plage de température pendant un laps de temps inférieur à 48 heures comprenant :
le chauffage de l'arbre à l'aide de lumière infrarouge émise par l'élément chauffant électrique à cristaux de carbone ;
la mesure d'une température de l'arbre à l'aide du capteur de température ;
la désactivation de l'élément chauffant électrique à cristaux de carbone lorsque la température mesurée a atteint une température souhaitée ; et
la réactivation de l'élément chauffant électrique à cristaux de carbone lorsque la température mesurée descend sous la température souhaitée ;
dans lequel la température souhaitée est d'au moins 39 degrés Celsius ; et
dans lequel l'arbre est un arbre du genre Aesculus, en particulier un arbre de l'espèce Aesculus hippocastanum, et/ou un arbre du genre Actinidia, en particulier un arbre de l'espèce Actinidia chinesis et/ou Actinidia deliciosa.

2. Procédé selon la revendication 1, dans lequel l'élément chauffant électrique à cristaux de carbone est composé d'une feuille comprenant des cristaux de carbone, dans lequel l'élément chauffant électrique à cristaux de carbone est conçu pour générer de la chaleur et un rayonnement infrarouge lorsqu'un courant électrique est fourni à celui-ci.

3. Procédé selon la revendication 1, dans lequel l'élément chauffant électrique à cristaux de carbone est composé d'une étoffe tissée comprenant des cristaux de carbone, dans lequel l'élément chauffant électrique à cristaux de carbone est conçu pour générer un rayonnement infrarouge ayant une longueur d'onde allant de 2 à 4 micromètres.

4. Procédé selon l'une quelconque des revendications précédentes, dans lequel ledit agencement d'un élément chauffant électrique à cristaux de carbone dans un voisinage de l'arbre comprend l'enveloppement de l'élément chauffant électrique à cristaux de carbone autour de partie(s) de l'arbre.

5. Procédé selon l'une quelconque des revendications précédentes, dans lequel le laps de temps pendant lequel la température est maintenue pour qu'elle soit au sein d'une plage de température est inférieur à 24 heures, plus préférablement inférieur à 12 heures, et le plus préférablement entre 2 et 12 heures.
